# EUROPÄISCHE PATENTSCHRIFT

(11) **EP 1 412 510 B1**
(45) Veröffentlichungstag und Bekanntmachung des Hinweises auf die Patenterteilung: **20.08.2014**
(21) Anmeldenummer: 02742733.5
(22) Anmeldetag: 08.05.2002
(51) Int. Cl.: C12N 15/86

(54) **AAV-VEKTOR-VERPACKUNGSPLASMIDE ZUR HELFERVIRUS-FREIEN HERSTELLUNG VON WTAAV-PARTIKELN ODER PSEUDOTYPISIERTEN AAV-PARTIKELN ÜBER EINZELTRANSFEKTION**
AAV VECTOR PACKAGING PLASMID FOR PRODUCING WTAAV PARTICLES OR PSEUDOTYPED AAV PARTICLES WITHOUT HELPER VIRUSES, BY MEANS OF A SINGLE TRANSFECTION
PLASMIDES D'ENCAPSIDATION DE VECTEURS AAV POUR LA PRODUCTION, SANS VIRUS ASSISTANT, DE PARTICULES D'AAV DE TYPE SAUVAGE OU DE PARTICULES D'AAV PSEUDOTYPEES PAR TRANSFECTION UNIQUE

(30) Priorität: 01.08.2001 DE 10137283
(43) Veröffentlichungstag der Anmeldung: 28.04.2004
(73) Patentinhaber: Deutsches Krebsforschungszentrum Stiftung des öffentlichen Rechts, 69120 Heidelberg (DE)
(72) Erfinder: GRIMM, Dirk, Palo Alto, CA 94303 (US); KLEINSCHMIDT, Jürgen, 69245 Bammental (DE)
(74) Vertreter: Grund, Martin
(86) Internationale Anmeldenummer: PCT/DE2002/001670
(87) Internationale Veröffentlichungsnummer: WO 2003/016521

(56) Entgegenhaltungen:
- WO-A-01/25253
- WO-A-95/06743
- WO-A-98/21345
- US-A- 5 965 441

## Beschreibung

Die vorliegende Erfindung betrifft AAV-Vektor-Verpackungsplasmide zur Helfervirus-freien Herstellung von (pseudotypisierten) AAV-Partikeln über Einzeltransfektion. Die AAV-Vektor-Verpackungsplasmide für die (pseudotypisierten) AAV-Partikel beinhalten folgende DNA-Sequenzen: (a) Ein *rep*-Gen von AAV, (b) ein *cap*-Gen von AAV, (c) AAV-Expressionsvektor-DNA-Sequenzen, und (d) alle weiteren Helfervirus-DNA-Sequenzen, die für eine Ausbildung von AAV-Partikeln notwendig sind. Die AAV-Vektor-Verpackungsplasmide für die Herstellung von wtAAV-Partikeln sind dadurch gekennzeichnet, dass sie (a) das komplette AAV-Genom sowie (b) alle Helfervirus-DNA-Sequenzen, die für eine Ausbildung von AAV-Partikeln notwendig sind, umfassen. Die vorliegende Erfindung betrifft ferner die Verwendung dieser AAV-Vektoren zur Herstellung von wtAAV-Partikeln oder pseudotypisierten AAV-Partikeln, insbesondere zur Gentherapie oder Tumortherapie.

AAVs sind einzelsträngige, zur Familie der Parvoviren gehörende DNA-Viren. Für ihre Replikation benötigen AAVs Helferviren, insbesondere Adenoviren oder Herpesviren. In Abwesenheit von Helferviren integrieren AAVs in das Wirtszellgenom, insbesondere an einer spezifischen Stelle von Chromosom 19. Das Genom von AAVs ist linear und weist eine Länge von ca. 4680 Nukleotiden auf. Dieses umfaßt zwei Leserahmen, die für ein strukturelles und ein nichtstrukturelles Gen codieren. Das strukturelle Gen wird als *cap*-Gen bezeichnet. Dieses steht unter der Kontrolle des P40-Promotors und kodiert für drei Capsid-Proteine. Das nicht-strukturelle Gen wird mit *rep*-Gen bezeichnet und kodiert für die Rep-Proteine Rep 78, Rep 68, Rep 52 und Rep 40. Die beiden ersteren werden unter der Kontrolle des P5 Promotors exprimiert, während die Expression von Rep 52 und Rep 40 unter der Kontrolle des P19-Promotors steht. Die Funktionen der Rep-Proteine liegen u.a. in der Regulation der Replikation und Transkription des AAV-Genoms.

AAVs werden schon seit einiger Zeit intensiv als mögliche Vektoren für die humane Gentherapie entwickelt und getestet. Unter den sechs verschiedenen Serotypen von AAV (AAV-1 bis AAV-6), die bisher kloniert und sequenziert wurden, stellt AAV-2 den am besten charakterisierten Serotyp dar und die meisten zur Zeit verwendeten Vektoren basieren auf AAV-2. In den letzten Jahren wurden jedoch auch Berichte über die Erzeugung und Evaluierung der anderen fünf AAV-Serotypen veröffentlicht. Es stellte sich heraus, daß die ITRs (inverted terminal repeats) an jedem Ende des AAV-Genoms die einzigen *cis*-Elemente sind, die für die Vermehrung (d.h. Exzision der viralen DNA vom Plasmid, Replikation und Verpackung der intermediären DNA-Sequenzen) von AAV-Vektoren (mit Hilfe des Helfervirus) erforderlich sind. Somit war es naheliegend, daß im Prinzip jede DNA, die von AAV-ITRs flankiert ist und hinsichtlich der Länge mit einem Wildtyp-Virusgenom vergleichbar ist, in AAV-Kapside in Gegenwart der *rep-* und *cap*-Genprodukte *in trans* sowie der Helfervirusfunktionen verpackt werden kann. In den meisten Fällen wurde für die Herstellung dieser Vektoren ein Verfahren angewandt, bei dem Helferviren verwendet werden müssen, d.h., die Zellen werden mit dem AAV-Vektor und Helferplasmiden cotransfiziert und danach mit dem Helfer-Adenovirus infiziert, was zu rekombinanten AAV-Vektoren führt, die allerdings mit Adenovirus kontaminiert sind. Eine weitere Strategie beruht auf einer Dreifach-Transfektion, bei der zusätzlich zur Vermeidung einer Kontamination mit Helferviren zur Bereitstellung von Helferfunktionen ein nicht-infektiöses adenovirales Plasmid verwendet wird.

Zusammengefaßt gibt es zur Zeit drei unterschiedliche Ansätze: (a) Cotransfektion von AAV-Helfersequenzen, die das rep- und *cap*-Gen des jeweiligen AAV-Serotyps zur Verfügung stellen, sowie den entsprechenden Vektorplasmiden von AAV-2, AAV-3, AAV-5 oder AAV-6, (b) Cotransfektion von AAV-2 Vektorplasmiden mit AAV-Helferplasmiden, die das *rep*-Gen von AAV-2 und das *cap*-Gen von AAV-1, AAV-3 oder AAV-5 tragen, und (c) Cotransfektion von AAV-2- Vektorplasmiden mit *rep-cap*-Genen von AAV-1, AAV-3, AAV-4 oder AAV-6. Die adenoviralen Helferfunktionen werden bei allen drei Ansätzen durch Infektion mit Adenoviren oder durch zusätzliche Transfektion von Plasmiden, die das adenovirale Genom tragen, zur Verfügung gestellt. Allerdings weisen alle bisherigen Ansätze bestimmte gravierende Nachteile auf. So müssen (a) entweder verschiedene Vektorplasmide für die Verpackung in verschiedene AAV-Serotypen verwendet werden, (b) die Vektorproduktion durch Tripelinfektionen ist umständlich und teuer und (c) bei der Doppeltransfektion und Infektion mit Adenoviren ergibt sich das Problem der Kontamination mit Adenoviren.

Das der vorliegenden Erfindung zugrunde liegende technische Problem war somit, ein Mittel zur Herstellung von wtAAV-Partikeln und pseudotypisierten AAV-Partikeln bereitzustellen, das die vorstehend diskutierten Nachteile nicht aufweist.

Die Lösung dieses technischen Problems erfolgt durch die Bereitstellung der in den Ansprüchen gekennzeichneten Ausführungsformen. Es wurde überraschenderweise festgestellt, daß das technische Problem durch die Verwendung eines AAV-Vektor-Verpackungsplasmids gelöst werden konnte, das die vollständigen Helferfunktionen für die Verpackung der Vektor-DNA in Kapside von AAV, z.B. AAV-1, 2, 3, 4, 5 oder 6, wobei AAV-2 bevorzugt ist, sowie - für eine Pseudotypisierung - AAV-Expressionsvektor-DNA-Sequenzen enthält. Der Hauptvorteil ist dabei die Vereinfachung der Herstellung von (pseudotypisierten) AAV-Partikeln, die nur eine Einzeltransfektion der gewünschten Zelle (mit dem erfindungsgemäßen AAV-Vektor-Verpackungsplasmid) benötigen, was einen geringeren zeitlichen Aufwand und Kostenaufwand zur Folge hat. Außerdem entfällt das bei einer Tripel- oder Doppeltransfektion auftauchende Problem, daß nicht alle Zellen mit allen Plasmiden transfiziert werden, was zu einer geringeren Vektorproduktion und einem hohen Anteil an leeren Kapsiden führt. Es zeigte sich, daß die Partikel-Ausbeute im Vergleich zu den auf z.B. Doppeltransfektion basierenden Verfahren bis zu einem Faktor 10 gesteigert werden konnte. Außerdem sind die mit den erfindungsgemäßen AAV-Vektor-Verpackungsplasmiden erzeugten Partikel frei von einer Kontamination mit Adenoviren. Bei diesem einfachen Verfahren wurde von dem in der deutschen Patentanmeldung 196 44 500.0-41 beschriebenen Helferplasmid pDG ausgegangen, das alle AAV-2 und adenoviralen Gene besitzt, deren Produkte für die Erzeugung von AAV-2-Vektoren erforderlich sind. Dazu wurden lediglich in die verkürzte E3-Region (*E3*) von pDG die entsprechenden AAV-Expressionsvektor-DNA-Sequenzen einkloniert. Außerdem wurde in das in dem nachstehenden Beispiel 1 beschriebenen AAV-Vektor-Verpackungsplasmid zwischen dem *cap*-Gen und dem Ad5-Gen für VA eine Expressionskassette für das "red fluorescent"-Protein kloniert, wodurch noch ein weiterer Vorteil erzielt wird, da so die Transfektionseffizienz des AAV-Vektor-Verpackungsplasmids leicht visuell überprüft werden kann.

Gegenstand der vorliegenden Erfindung ist somit ein AAV-Vektor-Verpackungsplasmid, das folgende DNA-Sequenzen umfaßt:
(a) Ein rep-Gen von AAV unter der Kontrolle eines Mouse Mammary Tumor Virus - Promoters, ein *cap*-Gen von AAV und AAV-Expressionsvektor-DNA-Sequenzen sowie (b) alle weiteren Helfervirus-DNA-Sequenzen, die für eine Ausbildung von AAV-Partikeln notwendig sind.

Das *rep*-Gen bzw. *cap*-Gen stammen von den Serotypen AAV-1, AAV-2, AAV-3, AAV-4, AAV-5 oder AAV-6, wobei die beiden Gene vom gleichen AAV-Serotyp oder unterschiedlichen Serotypen stammen können.

Der hier verwendete Ausdruck "Helfervirus-DNA-Sequenzen" betrifft alle DNA-Sequenzen eines Helfervirus, die zur Ausbildung von AAV-Partikeln notwendig sind. Solche DNA-Sequenzen stammen von Adenovirus 5. Die Sequenzen können das gesamte Virus-Genom oder Fragmente davon umfassen. Geeignete Helfervirus-DNA-Sequenzen als Ausgangsmaterial für die Herstellung der erfindungsgemäßen AAV-Vektoren sind z.B. in der deutschen Patentanmeldung 196 44 500.0-41 beschrieben und dazu zählen z.B. auch die in dieser Patentanmeldung offenbarten DNA-Sequenzen des Plasmids pTG 9585, das als Helfervirus-DNA-Sequenz die vollständige Adenovirus 5-Sequenz mit Ausnahme der E1-Region umfaßt. Das erfindungsgemäße AAV-Vektor-Verpackungsplasmid kann auch Helfervirus-DNA-Sequenzen enthalten, die sich von denen in pTG 9585 dadurch unterscheiden, daß sie eine Deletion im Strukturgen L1 der Ad5-Sequenz, insbesondere im Bereich der Nukleotide 16614-18669 aufweisen. Sequenzen für die rep- bzw. *cap*-Gene sind in der Literatur beschrieben (*rep*-Gene und cap-Gene von AAV-1, AAV-3, AAV-4, AAV-5 oder AAV-6 (AAV-1, Xiao et al., J. Virol. 73 (1999), 3994-4003; AAV-3, Muramatsu et al., Virol. 221 (1996), 208-217; AAV-4, Chiorini et al., J. Virol. (1997), 6823-6833; AAV-5, Bantel-Schaal et al., J. Virol. 73 (1999), 939-947; Chiorini et al., J. Virol. 73 (1999), 1303-1319; AAV-6, Rutledge et al., J. Virol. 72 (1998), 309-319).

Der hier verwendete Ausdruck "komplettes AAV-Genom" betrifft die Gesamtheit der für die Herstellung eines infektiösen AAV Virus notwendigen AAV Gene und umfaßt die AAV rep- und cap-Gene, sowie mindestens eine terminale Wiederholungssequenz und die zur Genexpression notwendigen Kontrollelemente, z.B. Promotoren, Splice-Stellen, Polyadenylierungssignale. Diese Kontrollelemente können gegen andere Kontrollelemente zur Genexpression ausgetauscht werden.

Allgemeine, auf dem Fachgebiet bekannte Verfahren können zur Konstruktion von AAV-Vektor-Verpackungsplasmiden, die die vorstehenden DNA-Sequenzen und gegebenenfalls weitere Sequenzen enthalten, verwendet werden. Zu diesen Verfahren zählen beispielsweise in vitro-Rekombinationstechniken, synthetische Verfahren, sowie in vivo-Rekombinationsverfahren, wie sie beispielsweise in Sambrook et al., Molecular Cloning: A Laboratory Manual, 2. Ausgabe, Cold Spring Harbor Laboratory Press, Cold Spring Harbor NY (1989))., beschrieben sind. Die Herstellung der erfindungsgemäßen AAV-Vektor-Verpackungsplasmide kann z.B. durch homologe Rekombination in Bakterien, wie sie bei Chartier et al., J.Virol, 70 (1996), 4805-4810 beschrieben ist, erfolgen. Beispielsweise kann als Grundgerüst für ein erfindungsgemäßes AAV-Vektor-Verpackungsplasmid das in der deutschen Patentanmeldung 196 44 500.0-41 beschriebene Plasmid pDG verwendet werden, bei dem z.B. in die *E3*-Region die AAV-Expressionsvektorsequenzen inseriert werden.

Geeignete AAV-Expressionsvektor-DNA-Sequenzen sind dem Fachmann bekannt (Samulski et al., J.Virol. 63 (1989), 3822-3828, Zolotukhin et al., J. Virol. 70 (1996), 4646-4653). Es handelt sich dabei um DNA-Sequenzen, die mindestens folgende DNA-Sequenzen umfassen: (a) die 5' ITR und 3'ITR eines AAV-2; (b) einen in Säugern aktiven konstitutiven oder induzierbaren Promotor und (c) ein Poly-Adenylierungssignal. Dabei umfaßt der Begriff "5' ITR" bzw. "3' ITR" alle "5'ITR"- bzw. "3'ITR"-Sequenzen, die dem Vektor die Replikation und Verpackung in Virus Partikeln sowie die Integration in das Wirtsgenom erlauben. Der hier verwendete Ausdruck "ein in Säugern aktiver konstitutiver oder induzierbarer Promotor" umfaßt alle Promotoren, die in Säugern die Transkription der gewünschten DNA-Sequenz erlauben, vor allem solche, die zu einer starken Expression führen, vorzugsweise heterologe Promotoren. Geeignete Promotoren sind dem Fachmann bekannt und umfassen beispielsweise die konstitutiven Promotoren CMV- und Cytokeratin K14-Promotoren oder die induzierbaren Promotoren MMTV-(Mouse Mammary Tumor Virus), Metallothionein- und Tetrazyklin-regulierbare Promotersysteme (Tet-on/-off). Die AAV-Expressionsvektor-DNA-Sequenzen des AAV-Vektor-Verpackungsplasmids können darüber hinaus das gewünschte, in den Säugerzellen zu exprimierende Gen, dessen Expression z.B. für eine Gentherapie wünschenswert ist, enthalten. Der Fachmann kann eine geeignete Stelle innerhalb der DNA-Sequenz des AAV-Vektors zur Insertion der AAV-Expressionsvektor-DNA-Sequenzen gemäß Routine-Experimenten bestimmen, wobei bei der Auswahl der Insertionsstelle darauf zu achten ist, daß sowohl die Funktionen des AAV-Vektors (z.B. hinsichtlich Verpackung) als auch die der AAV-Expressionsvektor-DNA-Sequenzen (z.B. hinsichtlich Exzision und Expression der Fremd-DNA) nicht wesentlich beeinträchtigt werden. Vorzugsweise erfolgt die Insertion der AAV-Expressionsvektor-DNA-Sequenzen in eine verkürzte E3-Region (*E3*).

Das erfindungsgemäße AAV-Vektor-Verpackungsplasmid enthält als Helfervirus-DNA-Sequenzen die Ad5-Gene E2A, E4 und VA, die z.B. von dem in der deutschen Patentanmeldung 196 44 500.0-41 beschriebenen Plasmid pDG stammen können und wobei diese unter der Kontrolle des jeweiligen ursprünglichen Promotors stehen oder unter der Kontrolle heterologer Promotoren.

Ferner kann das AAV-Vektor-Verpackungsplasmid ein Gen enthalten, das einen nachweisbaren phänotypischen Marker codiert, und somit das erfolgreiche Einschleusen des AAV-Vektor-Verpackungsplasmids in die Zielzelle nachgewiesen werden kann. In einer noch mehr bevorzugten Ausführungsform enthält das erfindungsgemäße AAV-Vektor-Verpackungsplasmid somit zusätzlich eine Expressionskassette zur Expression eines Markerproteins, vorzugsweise eines Fluoreszenzproteins. In diesem Zusammenhang bedeutet der Begriff "Expressionskassette" eine Kombination aus einem z.B. ein Fluoreszenzgen kodierenden Gen und einem geeigneten Promotor, unter dessen Kontrolle dieses Gen steht, sowie einem Polyadenylierungssignal. Dies erlaubt den einfachen Nachweis einer Transfektion der gewünschten Zielzelle. Beispiele für geeignete Fluoreszenzproteine kodierende Gene sind das rfp-(red), gfp-(green), cfp-(cyan) und yfp-(yellow) Gen, wobei rfp-(red) (Dsred-cDNA; Clontech) bevorzugt ist. Beispiele für geeignete Promotoren sind RSV (Rous Sarcoma-Virus)-Promotor, CMV (Cytomegalo-Virus)-Promotor und HSV (Herpes Simplex-Virus)-tk Promotor, wobei der RSV-Promotor bevorzugt ist. Diese Expressionskassette wird in das AAV-Vektor-Verpackungsplasmid an einer geeigneten Stelle, die der Fachmann leicht ermitteln kann, inseriert, vorzugsweise zwischen dem 3'-Ende des *cap*-Gens und dem Beginn des adenoviralen VA-Gens eingefügt, z.B in die ClaI-Schnittstelle. Diese ClaI-Schnittstelle ist in pDG vorhanden.

In einer weiteren besonders bevorzugten Ausführungsform betrifft die vorliegende Erfindung ein AAV-Vektor-Verpackungsplasmid, wobei die AAV-Expressionsvektor-DNA-Sequenzen eine HPV16-L1 kodierende DNA-Sequenz unter der Kontrolle eines CMV-Promotors enthalten. Ein solches mit pDS2-Lh1 bezeichnetes AAV-Vektor-Verpackungsplasmid wurde bei der DSMZ, Braunschweig, gemäß den Bestimmungen des Budapester Vertrags am 17. Juli 2001 unter DSM 14406 hinterlegt.

Ferner werden hierin wtAAV-Partikel offenbart, vorzugsweise wtAAV-2-Partikel, deren Genom ein komplettes AAV-Genom sowie alle weiteren Helfervirus-DNA-Sequenzen umfaßt, die für eine Ausbildung von AAV-Partikeln notwendig sind, sowie (pseudotypisierte) AAV-Partikel, deren Kapsidhülle von einem erfindungsgemäßen AAV-Vektor-Verpackungsplasmid kodiert ist und das AAV-Expressionsvektor-DNA-Sequenzen enthält.

Erfindungsgemäße AAV-Partikel können durch geeignete Verfahren erhalten werden, z.B. durch Einzeltransfektion von Säugerzellen, z.B. 293-Zellen oder 911-Zellen, mit einem erfindungsgemäßen AAV-Vektor-Verpackungsplasmid. Als Transfektionstechniken sind z.B. Elektroporation, Lipofektion und vorzugsweise Calciumphosphat-Präzipitation zu nennen. Der erzielbare Titer beträgt in der Regel zwischen 10 und 10 infektiöse Viren /ml.

Mit AAV-Partikeln, die mit einem erfindungsgemäßen AAV-Vektor-Verpackungsplasmid erzeugt worden sind, kann eine Gentherapie durchgeführt werden, wobei die Zellen durch Inkubation mit den Virus-Partikeln transduziert werden. Die Zellen können in einem Organismus vorliegen, wobei die zu infizierenden Zellen durch Nadel-Injektion, jet-injektion oder Partikel-Kanone erreicht werden können. Andererseits können die zu transduzierenden Zellen auch aus einem Organismus isoliert, außerhalb des Organismus infiziert und dann wieder in den Organismus zurückgeführt werden. Solche Zellen werden als autologe Zellen bezeichnet. Desweiteren können hinsichtlich des Organismus auch allogene Zellen zur Transduktion verwendet werden. Hierbei ist es günstig, wenn diese Zellen einem dem Organismus entsprechenden HLA-Typ angehören. Der Fachmann kennt Verfahren, Zellen einen bestimmten HLA-Typ zu verleihen. Die erfindungsgemäßen wtAAV-Partikel, vorzugsweise wtAAV-2-Partikel, sind auch zur adjuvanten Verwendung zur Chemotherapie, also zur Tumortherapie, von Nutzen.

Gegenstand der vorliegenden Erfindung ist somit auch ein Arzneimittel, das ein erfindungsgemäßes AAV-Vektor-Verpackungsplasmid bzw. ein AAV-Partikel enthält. Hierbei kann das Arzneimittel zusätzlich einen pharmazeutisch verträglichen Träger enthalten. Geeignete Träger und die Formulierung derartiger Arzneimittel sind dem Fachmann bekannt. Zu geeigneten Trägern zählen beispielsweise Phosphat-gepufferte Kochsalzlösungen, Wasser, Emulsionen, beispielsweise Öl/Wasser-Emulsionen, Netzmittel, sterile Lösungen etc. Die Art des Trägers hängt davon ab, wie das erfindungsgemäße AAV-Vektor-Verpackungsplasmid bzw. das AAV-Partikel verabreicht werden soll. Die geeignete Dosierung wird von dem behandelnden Arzt bestimmt und hängt von verschiedenen Faktoren ab, beispielsweise von dem Alter, dem Geschlecht, dem Gewicht des Patienten, dem Schweregrad der Erkrankung, der Art der Verabreichung etc.. Es hat sich dabei gezeigt, daß mit erfindungsgemäßen AAV-Vektor-Verpackungsplasmiden bzw. Partikeln hohe Transduktionsraten bei unterschiedlichsten Zellen, z.B. primären Zellen des Augen-Hornhautepithels oder Muskelzellen, erreicht werden können.

### Kurze Beschreibung der Figuren

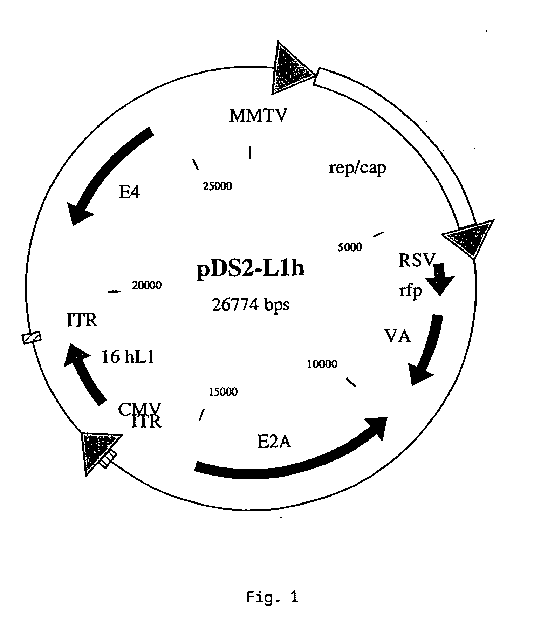

Figur 1: Physikalische Karte des AAV-Vektor-Verpackungsplasmids pDS2-L1h XYZ (ausgehend von pDG)
   Dargestellt ist das Plasmid pDS2-L1h, welches folgende Gene beinhaltet: das rep- und *cap*-Gen von AAV-2, die außerdem für eine AAV-2 Vektorproduktion essentiellen Gene von Adenovirus 5 *(E2A, E4 und VA*), sowie das Gen für das Rot-Fluoreszenzprotein
   (*rfp*). Die Expression des AAV-2 rep-Gens wird durch den heterologen *MMTV*-(Mouse Mammary Tumor Virus) Promotor gesteuert, die Expression des AAV-2 cap-Gens bzw. der adenoviralen Gene durch die authentischen viralen Promotoren von AAV-2 bzw. Adenovirus 5 (nicht dargestellt). Die Expression des rfp-Gens erfolgt unter der Kontrolle des *RSV*-(Rous Sarcoma Virus) Promotors. Weiterhin enthält pDS2-L1h AAV-2 Vektorsequenzen, bestehend aus zwei AAV-2 *ITR-*Regionen (invertierte terminale Wiederholungen, engl. inverted terminal repeats), welche das human-adaptierte Gen für das Kapsidprotein *L1* des humanen Papillomvirus Typ 16 flankieren. Die Expression des hL1-Gens erfolgt unter der Kontrolle des *CMV-*(Cytomegalovirus) Promotors.
Figur 2: Fluoresenzmarkierung transfizierter Zellen
   Gezeigt sind 293T-Zellen, welche mit dem in Figur 1 beschriebenen Plasmid pDS2-L1h transfiziert (6µg DNA, 4x10⁵ Zellen) und 48 Stunden nach Transfektion unter Fluoreszenzlicht (bei einer Wellenlänge von 558 nm zur Anregung der Fluoreszenz) fotografiert wurden.
Figur 3: Vergleich der Vektorproduktion nach Doppeltransfektion bzw. Einzeltransfektion mit einem erfindungsgemäßen AAV-Vektor-Verpackungsplasmid.
   Dargestellt sind die Ergebnisse der Titration der rekombinanten AAV-2 Partikel, welche aus den beiden alternativen Ansätzen, d.h. einer Einzel- oder Doppeltransfektion, gewonnen wurden. Die AAV-2 Vektoren wurden nach zwei Parametern quantifiziert: es wurde zum einen die Anzahl Genom-haltiger (voller) Partikel bestimmt ("Genome"), zum anderen die gesamte Anzahl an Virus-Partikeln ("Kapside"), d.h. die Summe aller vollen und leeren Partikel. Die Titration erfolgte mittels beschriebener Methoden (Grimm, et al., Hum. Gene Ther. 10, (1998), 2745-2760; Grimm, et al., Gene Ther. 6: (1999), 1322-1330). Zu erkennen ist, daß die Produktion der AAV-2 Vektoren mittels der Einzeltransfektion etwa 2,2-fach effizienter war.

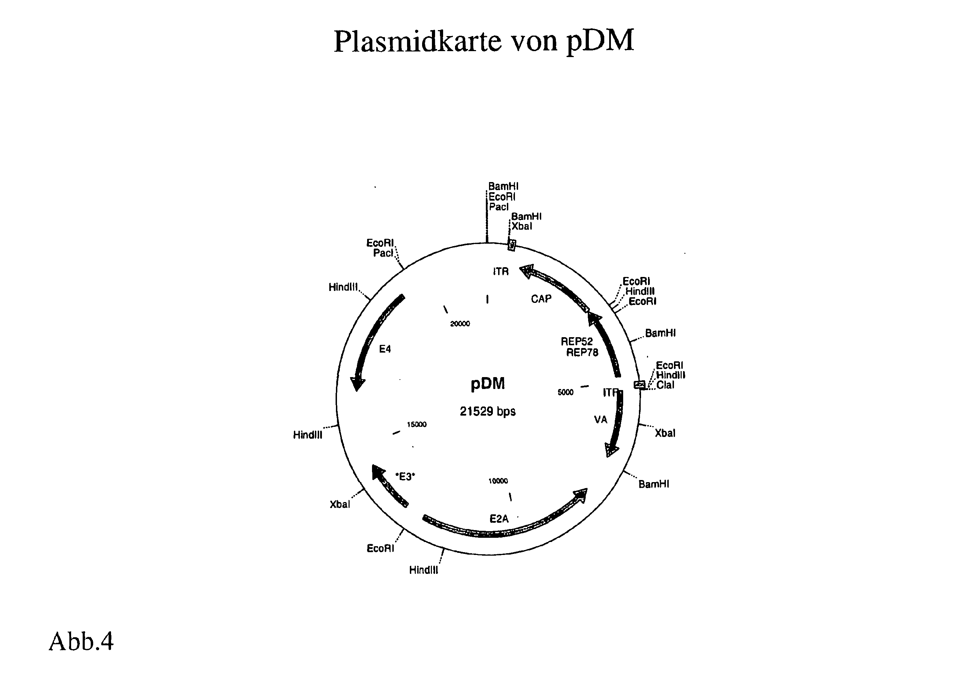

Figur 4: Plasmidkarte von pDM
   Das Plasmid pDM beinhaltet folgende Gene: die rep- und cap-Gene von AAV-2 flankiert von den beiden "inverted terminal repeats" (ITRs), sowie die für eine AAV-2 Virusproduktion essentiellen Gene von Adenovirus Typ5 (Ad5) E2A, E4 und VA. Die Expression der Gene erfolgt unter der Kontrolle der authentischen viralen Promotoren von AAV-2 bzw. von Ad5 (nicht dargestellt).

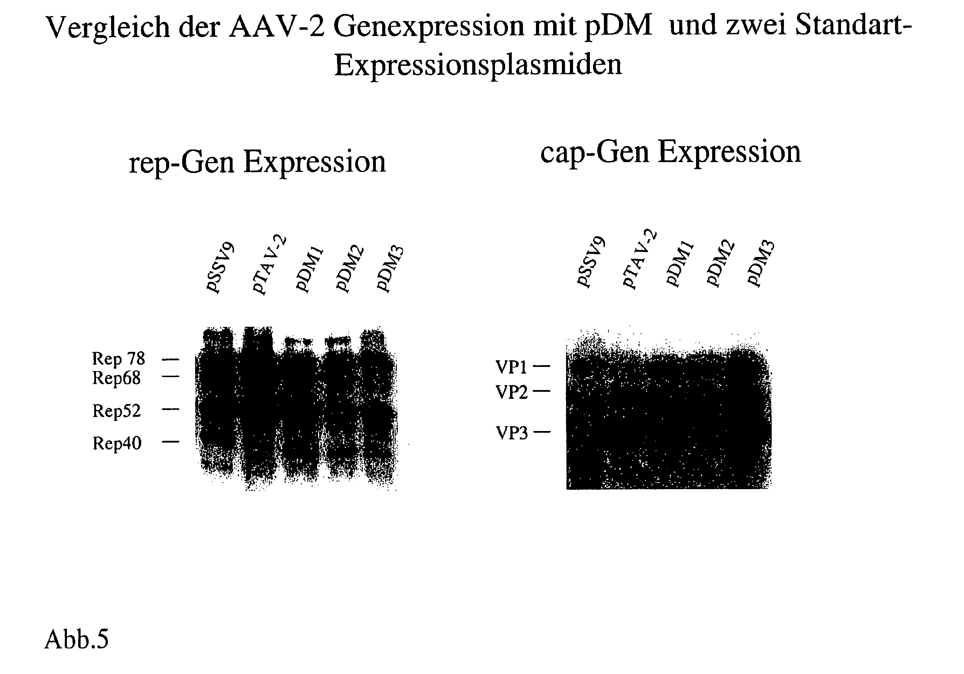

Figur 5: Vergleich der AAV-2 Genexpression mit pDM und zwei Standart-AAV-2 Expressionsplasmiden
   Dargestellt sind Western-Blot Analysen mit Antikörpern gegen Rep-Proteine (mAb 303.9) und VP-Proteine (mAb B1) von 293T Zellen, die mit AAV-2 Genom-Plasmiden transfiziert worden waren.

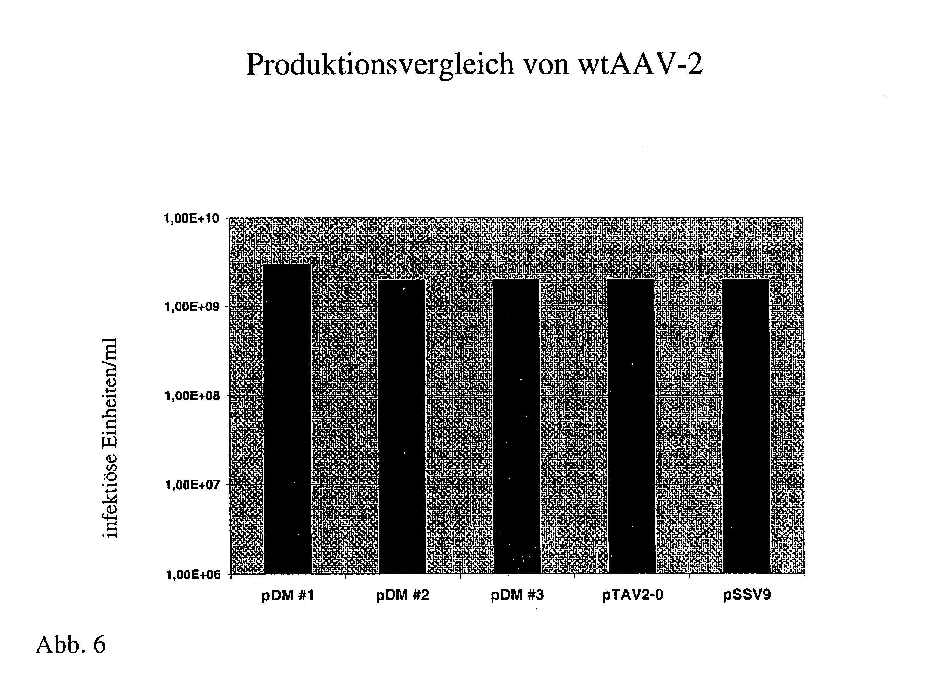

Figur 6: Vergleich der Produkion von wtAAV-2 mit Hilfe verschiedener AAV-2 Genom-Plasmide
   Siehe zur näheren Erläuterung Beispiel 6.

Die Erfindung wird durch die nachfolgenden Beispiele erläutert.

### Beispiel 1

### Herstellung des AAV-Vektor-Verpackungsplasmids pDS2-L1h

Zur Herstellung des AAV-Vektor-Verpackungsplasmids pDS2-L1h XYZ wurde von dem in der deutschen Patentanmeldung 196 44 500.0-41 beschriebenen Plasmid pDG ausgegangen. Dieses besitzt eine Gesamtlänge von 21846 Basenpaaren. Das in pDG enthaltene AAV-Genom besitzt einschließlich des den AAV-2 p5 Promotor ersetzenden MMTV-Promotors insgesamt eine Länge von 5044 Basenpaaren.

Die Konstruktion des Plasmides pDS2-L1h (S. Fig. 1) erfolgte durch homologe in vitro-Rekombination einer AAV-2 Vektor-DNA (s.u.) mit dem Plasmid pDF. Das Plasmid pDF ist ein Derivat von pDG und trägt zusätzlich das Gen für das Rot-Fluoreszenzprotein (rfp) unter der Kontrolle des RSV-Promotors. Die Vektor-DNA, bestehend aus den AAV-2 invertierten terminalen Wiederholungen (ITR), dem CMV-Promotor, dem L1-Gen des humanen Papillomvirus 16, sowie einer Poly-A-Region (abgeleitet vom Gen des Rinder-Wachstumshormons), wurde aus dem Plasmid pCMV-L1h isoliert (Leder et al., J. Virol. im Druck). Hierzu wurden durch Restriktion von pCMV-L1h drei Fragmente gewonnen; Fragment A entstand durch Restriktion mit Pstl und Notl, Fragment B wurde durch Restriktion mit Notl und Hindlll erhalten, und Fragment C durch Restriktion mit Hindlll und Pstl. Fragment A wurde dann in das Plasmid pBluescript (Stratagene) eingefügt, welches zuvor ebenfalls mit Pstl und Notl restringiert wurde. Die Fragmente B und C wurden gemeinsam in das mit Notl und Nsil linearisierte Plasmid pSL1180 (Pharmacia) einkloniert. Danach konnten die ursprünglichen Fragmente des L1h-Vektors (A, B und C) durch Restriktion mit Clal und Notl (Fragment A aus pBluescript) bzw. Notl und Nhel (Fragment B und C aus pSL1180) wieder isoliert werden. Die dabei erhaltenen zwei Fragmente (A' und BC') wurden dann gemeinsam in das Plasmid pBSΔE3UFΔ einkloniert. Dieses Plasmid basiert auf dem Plasmid pBSΔE3 (Grimm, D. 1998, Entwicklung und Anwendung neuartiger Methoden zur Produktion, Reinigung und Titration rekombinanter Adenoassoziierter Viren des Typ 2. Dissertation, Universität Heidelberg), welches ein 8471 Basenpaar großes Bgl 1-Fragment (Basenpaar 24876-35442 bezogen auf Wildtyp Adenovirus 5) aus Adenovirus d1324 beinhaltet (D1324 ist eine Mutante von Adenovirus 5, bei der durch Restriktion mit Xbal eine 1878 Basenpaar große Deletion innerhalb der E3-Region erzeugt wurde). in pBSΔE3 wurde zunächst eine AAV-2 Vektor-DNA eingefügt, welche von dem Plasmid pTR-UF3 (Zolotukhin et al., J. virol. 70, (1996), 4646-4654) abgeleitet war. Hierzu wurde aus pTR-UF3 durch partielle Restriktion mit Pstl ein Fragment isoliert und in das mit Pstl linearisierte Plasmid pSL1180 einkloniert. Danach wurde die AAV-2 Vektor-DNA aus dem entstandenen Plasmid als Nhel/Spel-Fragment isoliert und in das mit Xbal restringierte Plasmid pBSΔE3 eingefügt. Schließlich wurde das hierbei erzeugte Plasmid mit Clal und Xbal geschnitten, um die oben beschriebenen Fragmente A' und BC' des L1h-Vektors einklonieren zu können. Aus dem resultierenden Plasmid pBSΔE3UF wurde dann durch Restriktion mit Pacl ein Fragment gewonnen, welches die L1h-Vektor-DNA, sowie flankierende Sequenzen des d1324-Adenovirus enthielt. Dieses Fragment wurde durch Transformation gemeinsam mit dem mit Ndel linearisierten Plasmid pDF in E. coli BJ5183 (Chartier et al., J. Virol 70, (1986), 4805-4810) eingebracht. Die in den Bakterien erfolgte homologe Rekombination der beiden Fragmente resultierte in dem Plasmid pDS2-L1h.

### Beispiel 2

### Fluoreszenzmarkierung transfizierter Zellen

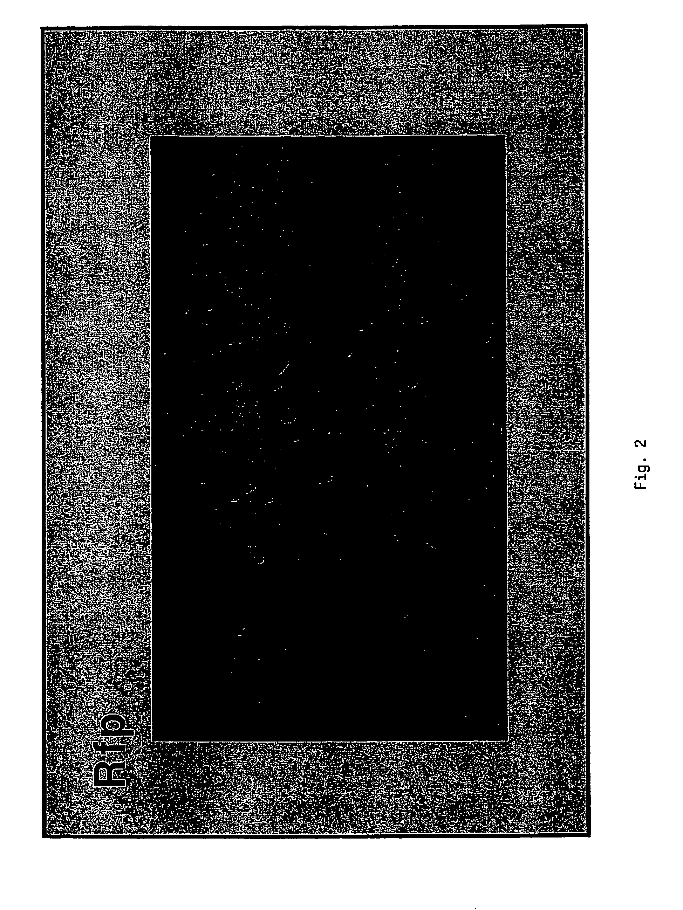

Das Plasmid pDS2-L1h trägt das Gen für das Rot-Fluoreszenzprotein unter der Kontrolle des RSV-Promotors; eine Transfektion von Zellen mit pDS2-L1h führt demnach zur Expression des Rot-Fluoreszenzproteins, welches nach Anregung bei geeigneter Wellenlänge (558nm) rot-orange fluoresziert. Eine maximale Stärke der Expression des Rot-Fluoreszenzproteins bzw. eine maximale Intensität der Fluoreszenz wird 1-2 Tage nach Beginn der Transfektion beobachtet (vgl. Fig. 2). Da jede bei der Transfektion von pDS2-L1h getroffene Zelle das Rot-Fluoreszenzprotein exprimiert und somit nach Anregung fluoresziert, erlaubt dies eine schnelle und einfache Überprüfung der Effizienz der Plasmid-Transfektion, welche ein wichtiger Parameter bei der Produktion von AAV-2 Vektoren ist.

### Beispiel 3

### Vergleich der Vektorproduktion nach Doppel- bzw. Einzeltransfektion mit dem gemäß Beispiel 1 hergestellten AAV-Vektor-Verpackungsplasmid

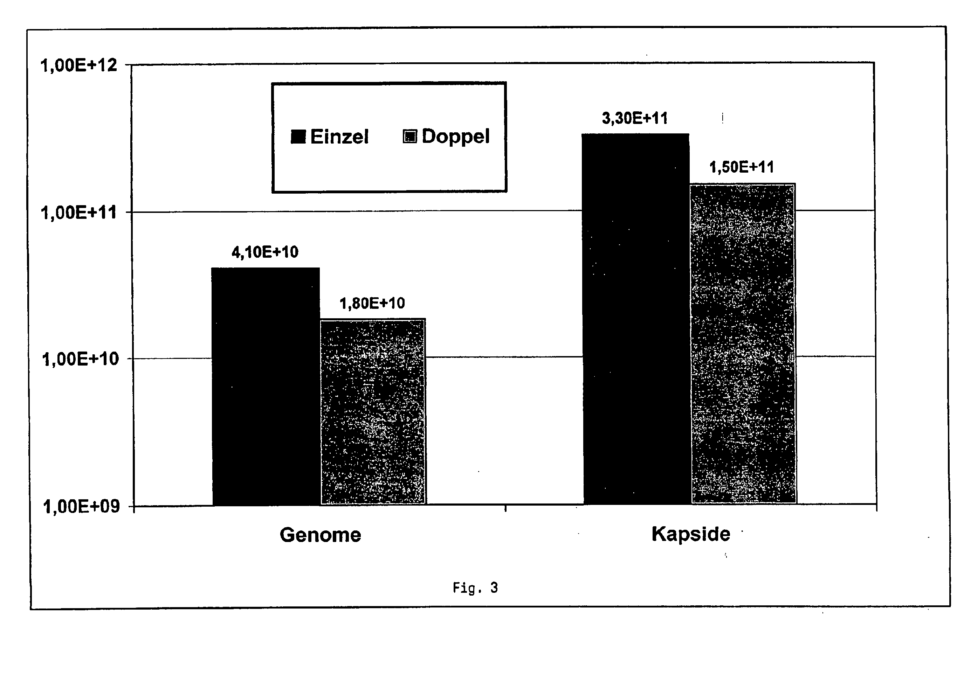

Das Plasmid pDS2-L1h trägt alle Gene bzw. Komponenten zur Herstellung von AAV-2 Vektoren, welche das Gen für das Kapsidgen L1 des humanen Papillomvirus Typ 16 beinhalten. Eine Transfektion von 293T-Zellen mit pDS2-L1h resultiert demnach in der Produktion solcher Vektoren. Konkret wurde folgender Ansatz erprobt: 4x10⁶ 293T-Zellen (in einer 15cm-Kulturschale) wurden mit 90µg pDS2-L1h transfiziert und danach 48 Stunden bei 37°C und 5 % CO2 inkubiert. Dann wurden die Zellen geerntet und gemäß einem Standardprotokoll (Hauswirth et al., Methods Enzymol. 316, (2000), 743-761) aufgeschlossen, um die produzierten AAV-2 Vektoren freizusetzen. In einem zweiten Ansatz wurden die Vektoren mittels einer Doppeltransfektion erzeugt. Hierzu wurden 293T-Zellen unter gleichen Bedingungen transfiziert, diesmal jedoch mit 70 µg pDF und 18 µg pCMV-L1h. Die Zellernte und die Freisetzung der Viren erfolgte wie oben beschrieben. Typische Ergebnisse solcher Einzel-bzw. Doppeltransfektions-Ansätze sind in Figur 3 dargestellt.

### Beispiel 4

### Herstellung des Plasmids pDM und Helfervirus-freie Produktion von wtAAV-2 Virus

Das Plasmid pDM wurde aus dem Plasmid pDG (Grimm et al., Human Gene Therapy 9, 2745-2760) abgeleitet. Die darin enthaltenen AAV-2 rep- und cap- Gene wurden durch Restriktion mit ClaI-NdeI, sowie mit XbaI-NdeI entfernt. Es entstehen dadurch zwei pDG-Fragmente, die die pDG Helfersequenzen ohne die AAV-2 rep-und cap-Gene enthalten. Das komplette AAV-2 Genom wurde aus dem Plasmid pTAV2-0 (Heilbronn et al., J. Virol. 64 (1990), 3012-3018) durch Restriktion mit XbaI-ClaI isoliert und mit den beiden pDG-Fragmenten (ClaI-NdeI, sowie XbaI-NdeI) in einer 3-Fragmente-Ligation zu pDM (Figur 4) ligiert. Das Plasmid pDM wurde unter der Nr. DSM 14927 am 10.4.2002 bei der DSMZ hinterlegt.

Eine Expressionskontrolle nach transienter Transfektion zeigte, daß mit Hilfe des pDM-Plasmids die Rep- und VP-Proteine in vergleichbaren Mengen zu dem Ausgangsplasmid pATV2-0, bzw. einem weiteren Referenz-Plasmid (pSSV9, identisch mit psub 201; Samulski et al., J. Virol. 61 (1987), 3096- 3101) exprimiert wurden (Figur 5). Ebenso ist die wtAAV-2 Virusproduktion mit Hilfe des pDM Plasmids ebenso effizient wie die Produktion mit Hilfe der Plasmide pTAV2-0 bzw. pSSV9, die zusätzlich eine Infektion mit Ad5 benötigen (Figur 6). Der Vorteil des pDM Plasmids zur Erzeugung von wtAAV-2 Viren liegt darin, dass bei gleicher Effizienz wtAAV-2 Viren ohne Helferviren und damit ohne Helferviruskontamination produziert werden können.

### Beispiel 5

### Vergleich der AAV-2 Genexpression mit pDM und zwei Standart-AAV-2 Expressionsplasmiden

293T Zellen wurden unter Standard-Bedingungen in Petri-Schalen (10 cm Durchmesser) kultiviert und bei ca. 60% Konfluenz mit jeweils 12 µg DNA nach der Methode von Chen und Okayama (1988, Bio Techniques 6, 632-38) transfiziert. Im Falle der Plasmide pSSV9 (entspricht psub 201; Samulski et al. (1987)) und pTAV2-0 (Heilbronn et al. J. Virol. 64, (1990)) 3012- 3018) wurden die Zellen zusätzlich mit Ad5 (MOI: 5) überinfiziert. Dies ist nach Transfektion mit den Plasmiden pDM1, 2 und 3 (1-3 stellen verschiedene Klone des gleichen Konstruktes dar) nicht notwendig, da die Plasmide das AAV-2 Genom und die Gene für die adenoviralen Helferfunktionen tragen (siehe Figur 4). Die Zellen wurden 48 Stunden nach Transfektion abgeschabt, sedimentiert (300xg für 5 Minuten), einmal mit PBS gewaschen und mit 500 µl 2x SDS-Probenpuffer versetzt. Danach wurden sie durch Erhitzen auf 100°C für 5 Minuten lysiert und für 20 Sekunden mit 80% Leistung von außen beschallt. Zelltrümmer wurden 2 Minuten bei 16500xg sedimentiert und 15µl der Überstände wurden zur SDS-Polyacrylamid-Gelelektrophorese verwendet. Dargestellt sind Western-Blot Analysen mit Antikörpern gegen Rep-Proteine (mAb 303.9 Wistuba et al. J.Virol. 69, 5311-5319 (1995)) und VP-Proteine (mAb B1 9 Wistuba et al., J.Virol. 69, 5311-5319 (1995)) von 293T Zellen, die mit AAV-2 Genom-Plasmiden transfiziert worden waren).

### Beispiel 6

### Vergleich der Produkion von wtAAV-2 mit Hilfe verschiedener AAV-2 Genom-Plasmide

Es wurde die wtAAV-2 Produktion nach Transfektion von 293T Zellen mit herkömmlichen AAV-2 Genom-Plasmiden (pSSV9 und pTAV2-0) und Überinfektion mit einem Helfervirus (Ad5; MOI:5), bzw. nach Transfektion mit dem Plasmid pDM ohne Helfervirus-Infektion verglichen (bezüglich Transfektion und Züchtung der Zellen siehe Beispiel 3). In Figur 6 sind Ergebnisse der Titrationen der AAV-2 Viren, die mit den entsprechenden Methoden gewonnen wurden, dargestellt. Die Titration erfolgte über eine Endpunktbestimmung durch den Nachweis der erfolgreichen Infektion von HeLa Zellen mittels eines AAV-2 Rep-Antikörpers (mAb 76/3 Wistuba et al, J.Virol. 69, 5311-5319 (1995)). Die HeLa Zellen wurden nach serieller Verdünnung der AAV-2 Virus Stocklösungen mit Ad5 und AAV-2 koinfiziert. Man sieht, daß die helfervirus-freie Produktion von wtAAV-2 genau so effizient ist wie die Helfervirus-unterstützte Produktion.

## Patentansprüche

1. AAV-Vektor-Verpackungsplasmid, das folgende DNA-Sequenzen umfasst:
(a) Ein AAV *rep*-Gen unter der Kontrolle eines Mouse Mammary Tumor Virus (MMTV)-Promoters, wobei das AAV *rep*-Gen ausgewählt ist aus der Gruppe bestehend aus AAV-1 *rep*-Gen wie in Xiao et al., J. Virol. 73 (1999), 3994-4003 beschrieben, AAV-2 *rep-*Gen wie in DSM 14406 hinterlegt enthalten, AAV-3 *rep*-Gen wie in Muramatsu et al., Virol. 221 (1996), 208-217 beschrieben; AAV-4 *rep*-Gen wie in Chiorini et al., J. Virol. (1997), 6823-6833 beschrieben; AAV-5 *rep*-Gen wie in Chiorini et al., J. Virol. 73 (1999), 1303-1319 beschrieben; AAV-6 *rep*-Gen wie in Rutledge et al., J. Virol. 72 (1998), 309-319 beschrieben,
ein AAV *cap*-Gen und
AAV-Expressionsvektor-DNA-Sequenzen, wobei die AAV-Expressionsvektor-DNA-Sequenzen DNA-Sequenzen sind, die mindestens folgende DNA-Sequenzen umfassen: (i) die 5' ITR und 3' ITR eines AAV-2; (ii) einen in Säugern aktiven konstitutiven oder induzierbaren Promotor und (iü) ein Poly-Adenylierungssignal, sowie
(b) alle weiteren Helfervirus-DNA-Sequenzen, die für eine Ausbildung von AAV-Partikeln notwendig sind, wobei die Helfervirus-DNA-Sequenzen die Adenovirus 5-Gene für E2A, E4 und VA sind.

2. AAV-Vektor-Verpackungsplasmid nach Anspruch 1, das zusätzlich eine Expressionskassette zur Expression eines Fluoreszenzproteins enthält.

3. AAV-Vektor-Verpackungsplasmid nach Anspruch 2, wobei die Expressionskassette zur Expression des Fluoreszenzproteins zwischen dem 3'-Ende des *cap*-Gens und dem 5'-Ende des Ad5-Gens für VA lokalisiert ist.

4. AAV-Vektor-Verpackungsplasmid nach Anspruch 2 oder 3, wobei das Fluoreszenzprotein das "red fluorescent" Protein ist.

5. AAV-Vektor-Verpackungsplasmid nach einem der Ansprüche 2 bis 4, wobei das Fluoreszenzprotein mit einem RSV-Promotor funktionell verknüpft ist.

6. AAV-Vektor-Verpackungsplasmid nach einem der Ansprüche 1 bis 5, wobei die AAV-Expressionsvektor-DNA-Sequenzen eine für das HPV 16-L1 Protein kodierende DNA-Sequenz unter der Kontrolle eines CMV-Promotors enthalten.

7. Verwendung eines AAV-Vektor-Verpackungsplasmids nach einem der Ansprüche 1-6 zur Herstellung von pseudotypisierten AAV-Partikeln, insbesondere zur Gentherapie oder Tumortherapie.

8. Verfahren zur Herstellung eines pseudotypisierten AAV-Partikels, **dadurch gekennzeichnet, dass** man Säugerzellen mit einem AAV-Vektor-Verpackungsplasmid nach einem der Ansprüche 1 bis 6 transfiziert und nach Züchtung der Zellen das AAV-Partikel aus den Säugerzellen oder dem Medium isoliert.

## Claims

1. An AAV vector packaging plasmid, comprising the following DNA sequences:
(a) An AAV *rep* gene under the control of a mouse mammary tumor virus (MMTV) promoter, wherein the AAV *rep* gene is selected from the group consisting of AAV-1 *rep* gene as described in Xiao et al., J. Virol. 73 (1999), 3994-4003, AAV-2 *rep* gene as contained in deposited DSM 14406, AAV-3 *rep* gene as described in Muramatsu et al., Virol. 221 (1996), 208-217; AAV-4 *rep* gene as described in Chiorini et al., J. Virol. (1997), 6823-6833; AAV-5 *rep* gene as described in Chiorini et al., J. Virol. 73 (1999), 1303-1319; AAV-6 *rep* gene as described in Rutledge et al., J. Virol. 72 (1998), 309-319,
an AAV- *cap* gene, and
AAV expression vector DNA sequences, wherein the AAV expression vector DNA sequences are DNA sequences comprising at least the following DNA sequences: (i) the 5'ITR and 3'ITR of a AAV-2; (ii) a constitutive or inducible promoter that is active in mammals and (iii) a polyadenylation signal; and
(b) all further helper virus DNA sequences being necessary for a formation of AAV particles, wherein the helper virus DNA sequences are the adenovirus 5 genes for E2A, E4 and VA.

2. An AAV vector packaging plasmid according to claim 1, additionally containing the expression cassette for expression of a fluorescent protein.

3. An AAV vector packaging plasmid according to claim 2, wherein the expression cassette for expression of the fluorescent protein is located between the 3' end of the *cap* gene and the 5' end of the Ad5 gene for VA.

4. An AAV vector packaging plasmid according to claim 2 or 3, wherein the fluorescent protein is the "red fluorescent" protein.

5. An AAV vector packaging plasmid according to any one of claims 2 to 4, wherein the fluorescent protein is operably linked to a RSV promoter.

6. An AAV vector packaging plasmid according to any one of claims 1 to 5, wherein the AAV expression vector DNA sequences contain a DNA sequence encoding the HPV 16-L1 protein under the control of a CMV promoter.

7. The use of an AAV vector packaging plasmid according to any one of claims 1-6 for producing pseudotyped AAV particles, particularly for gene therapy or tumor therapy.

8. A method for producing a pseudotyped AAV particle **characterized in that** mammalian cells are transfected with an AAV vector packaging plasmid according to any one of claims 1 to 6, and after growing of the cells the AAV particle is isolated from the mammalian cells or the medium.

## Revendications

1. Plasmide d'encapsidation de vecteur AAV, qui comprend les séquences d'ADN suivantes :
(a) un gène *rep* d'AAV sous le contrôle d'un promoteur de virus de tumeur mammaire de souris (MMTV), le gène rep d'AAV étant choisi dans le groupe consistant en le gène *rep* d'AAV-1 tel que décrit dans Xiao et al., J. Virol. 73 (1999), 3994-4003, le gène *rep* d'AAV2 tel que déposé sous DSM 14406, le gène *rep* d'AAV-3 tel que décrit dans Muramatsu et al., Virol. 221 (1996), 208-217 ; le gène *rep* d'AAV-4 tel que décrit dans Chiorini et al., J. Virol. (1997), 6823-6833 ; le gène *rep* d'AAV-5 tel que décrit dans Chiorini et al., J. Virol. 73 (1999), 1303-1319 ; le gène *rep* d'AAV-6 tel que décrit dans Rutledge et al., J. Virol. 72 (1998), 309-319,
un gène *cap* d'AAV et
des séquences d'ADN de vecteur d'expression d'AAV, les séquences d'ADN de vecteur d'expression d'AAV étant des séquences d'ADN qui comprennent au moins des séquences d'ADN suivantes : (i) les ITR en 5' et ITR en 3' d'un AAV-2 ; (ii) un promoteur constitutif ou inductible actif dans des mammifères et (iii) un signal de polyadénylation, ainsi que
(b) toutes les autres séquences d'ADN de virus assistant qui sont nécessaires pour une formation de particules d'AAV, les séquences d'ADN de virus assistant étant les gènes d'adénovirus 5 d'E2A, E4 et VA.

2. Plasmide d'encapsidation de vecteur AAV selon la revendication 1, qui contient en outre une cassette d'expression pour l'expression d'une protéine fluorescente.

3. Plasmide d'encapsidation de vecteur AAV selon la revendication 2, la cassette d'expression pour l'expression de la protéine fluorescente étant localisée entre l'extrémité 3' du gène *cap* et l'extrémité 5' du gène Ad5 de VA.

4. Plasmide d'encapsidation de vecteur AAV selon la revendication 2 ou 3, la protéine fluorescente étant la protéine « red fluorescent ».

5. Plasmide d'encapsidation de vecteur AAV selon l'une des revendications 2 à 4, la protéine fluorescente étant reliée fonctionnellement à un promoteur RSV.

6. Plasmide d'encapsidation de vecteur AAV selon l'une des revendications 1 à 5, les séquences d'ADN de vecteur d'expression d'AAV contenant une séquence d'ADN codant pour la protéine 16-L1 de HPV sous le contrôle d'un promoteur CMV.

7. Utilisation d'un plasmide d'encapsidation de vecteur AAV selon l'une des revendications 1 à 6, pour la production de particules d'AAV pseudotypées, en particulier pour la thérapie génique ou la thérapie de tumeurs.

8. Procédé de production d'une particule d'AAV pseudotypée, **caractérisé en ce que** l'on transfecte des cellules de mammifère avec un plasmide d'encapsidation de vecteur AAV selon l'une des revendications 1 à 6 et après culture des cellules on isole la particule d'AAV des cellules de mammifère ou du milieu.
